# FASCICULE DE BREVET EUROPEEN

(11) **EP 0 588 866 B1**
(45) Date de publication et mention de la délivrance du brevet: **16.08.1995**
(21) Numéro de dépôt: 92911794.3
(22) Date de dépôt: 05.06.1992
(51) Int. Cl.: G01N 13/00

(54) **DISPOSITIF DE CARACTERISATION DES PROPRIETES MOUSSANTES D'UN PRODUIT AU MOINS PARTIELLEMENT SOLUBLE**
VORRICHTUNG ZUM KENNZEICHEN DER SCHAUMEIGENSCHAFTEN EINES MINDESTENSZUM TEIL LOESBAREN PRODUKTES
DEVICE FOR DETERMINING THE FOAMING PROPERTIES OF AN AT LEAST PARTIALLY SOLUBLE MATERIAL

(30) Priorité: 11.06.1991 FR 9107269
(43) Date de publication de la demande: 30.03.1994
(73) Titulaire: INSTITUT NATIONAL DE LA RECHERCHE AGRONOMIQUE, F-75341 Paris Cédex 07 (FR)
(72) Inventeur: LOISEL, William, F-44340 Nantes (FR)
(74) Mandataire: Michelet, Alain
(86) Numéro de dépôt international: FR9200509
(87) Numéro de publication internationale: WO9222799

(56) Documents cités:
- EP-A- 0 252 738
- DE-A- 2 551 260
- FR-A- 1 476 857
- GB-A- 2 158 574
- US-A- 3 151 061
- US-A- 5 003 488
- JOURNAL OF FOOD SCIENCE vol. 48, 1 July 1983, pages 62 - 65; A.KATO ET AL.:'DETERMINATION OF FOAMING PROPERTIES'

## Description

La présente invention a pour objet un dispositif apte à caractériser les propriétés moussantes (capacité moussante et stabilité de la mousse) de produits en solution ou en suspension.

Dans les domaines de la cosmétologie ou de l'agroalimentaire notamment, on cherche à définir de la façon la plus fine possible les propriétés moussantes de certains produits destinés à être incorporés dans des préparations diverses, alimentaires ou non, caractérisées par une texture aérée ou foisonnée, du type savons, shampoings, mousses de foies... A partir d'une suspension ou d'une solution du produit à tester, une mousse est caractérisée par ses conditions de formation et de dégradation ; les propriétés moussantes sont ainsi déterminées par trois facteurs à savoir :
- la cinétique de formation de la mousse,
- la capacité moussante, qui correspond à la mesure du volume de mousse formée après introduction d'une quantité déterminée de gaz dans la solution et
- la stabilité de la mousse, qui correspond au taux de diminution du volume de mousse dans le temps.

Les méthodes visuelles de détermination et de mesure de ces facteurs, par l'évaluation du volume de mousse, ne donnent pas entière satisfaction du fait, d'une part, de la relative imprécision des mesures et, d'autre part, du fait que ce type de mesure ne reflète pas les facteurs qualitatifs des mousses tels que l'épaisseur des parois ou la taille des bulles.

En outre, différents facteurs tels que la concentration, le pH, la température et les méthodes de production de mousse influencent les propriétés moussantes des produits en solution ou en suspension.

Etant donné la volonté, tant au niveau des industriels que des laboratoires de recherche, de disposer de procédures normalisées pour la mesure des propriétés moussantes des protéines notamment, on a cherché à estimer les propriétés moussantes par la mesure de constantes physiques déterminant les caractéristiques des mousses. Il s'est avéré que ces caractéristiques pouvaient être approchées par la mesure de la conductivité des mousses (Journal of Food Science-Volume 48, 1983 pages 62 à 65). Cet article décrit un dispositif qui permet de caractériser les propriétés moussantes de produits au moins partiellement solubles, à partir de la mesure de la conductivité de la mousse formée. Le dispositif est constitué d'une colonne de verre, disposée verticalement et obturée, à sa base, par un filtre de verre sur lequel est disposée la solution à analyser. Une arrivée de gaz connectée au niveau de la partie inférieure de la colonne permet de générer un flux gazeux à travers le filtre de verre pour faire mousser la solution. Une cellule de mesure de la conductivité est disposée sur la hauteur de la colonne de façon à permettre la détermination de la conductivité de la mousse formée.

Les études réalisées à partir de ce dispositif montrent une corrélation étroite entre la conductivité initiale des mousses et la capacité moussante (capacité moussante = f(Ci)), ainsi qu'entre cette conductivité et la stabilité de la mousse (stabilité de la mousse = f (Co x Δ t/ Δ C)) où :
Δ C = changement de conductivité
Δ t = intervalle de temps
Co = conductivité à t = 0, obtenue par extrapolation de la deuxième partie de la courbe C = f(t).

Ce type de mesure permet l'observation de différences dans les propriétés moussantes qui ne peuvent être détectées à partir du simple changement de volume dans la mousse. Cependant, ce type de mesure ne rend compte des propriétés moussantes du produit analysé que de façon indirecte, avec tous les risques d'erreurs liés au système de corrélation. De plus, même si il permet de standardiser la détermination des propriétés moussantes par des contraintes importantes, cet appareil est limité quant au panel de caractéristiques pouvant être déduites de la mousse en formation ou en cours de dégradation. En plus, la mesure ne porte que sur une fraction de la mousse.

Les documents EP-A-0 252 738, GB-A-2 158 574 et FR-A-1 476 857 décrivent d'autres types de dispositifs dont le but est de caractériser les propriétés moussantes de produits. Comme pour l'appareil précédemment analysé, ces dispositifs ne permettent pas de ressortir plusieurs caractéristiques liées aux propriétés moussantes des produits analysés ; la caractérisation de ces propriétés n'est pas suffisante et les dispositifs correspondants ne donnent pas entière satisfaction.

La présente invention a pour but de remédier à ces inconvénients en proposant un dispositif de caractérisation des propriétés moussantes de produits en solution ou en suspension, susceptible de mesurer de façon automatique tout un ensemble de caractéristiques des mousses en vue d'affiner la caractérisation des propriétés moussantes du produit analysé.

Les mesures absolues retirées de la mousse formée ou en cours de dégradation permettent de caractériser en partie les propriétés moussantes du produit testé ; en outre, à partir de ces mesures absolues, il est possible de sortir des mesures calculées permettant d'affiner la caractérisation et de définir plus précisément les aspects qualitatifs et quantitatifs des mousses.

L'invention a pour objet un dispositif de caractérisation des propriétés moussantes d'un produit au moins partiellement soluble, du type décrit ci-dessus, c'est-à-dire comportant une colonne d'analyse transparente, munie, à sa base, d'un moyen en forme de filtre poreux par exemple, qui permet l'incorporation d'un flux de gaz et sa répartition de façon homogène pour faire mousser la solution placée dans le bas de la colonne d'analyse. Selon l'invention, le dispositif comporte un système de mesure automatique de la hauteur de mousse formée dans la colonne et un système de mesure automatique du volume de solution utilisé pour former cette mousse. A partir de la mesure du volume de mousse et du volume de liquide dans la mousse, on peut très simplement calculer la densité physique de la mousse (rapport volume de mousse/volume de liquide) ; ce calcul est de préférence réalisé de façon automatique.

La mesure de la hauteur de mousse est avantageusement obtenue au moyen d'une caméra linéaire disposée face à la colonne d'analyse. Cette caméra peut en outre être utilisée simultanément pour mesurer l'opacité de la mousse.

Le système de mesure du volume de solution utilisé pour former la mousse consiste de façon avantageuse en une paire d'électrodes, par exemple en platine, disposées dans le fond de la colonne et reliées à un conductimètre.

Selon une autre caractéristique du dispositif, la colonne d'analyse comporte, à sa partie inférieure, un moyen de distribution du flux gazeux en forme de vanne. La fermeture de cette vanne permet l'injection de gaz à travers le filtre et son ouverture réalise la connexion avec un dispositif en forme de colonne de liquide qui permet d'assurer une contrepression au travers du filtre qui évite la percolation et le drainage de la solution. La hauteur de la colonne de liquide est réglée en fonction de la contrepression désirée.

Selon une autre caractéristique de l'invention, le dispositif comporte un système de mesure de la quantité de gaz insufflé. On peut également prévoir un système de mesure de la conductivité de la mousse, constitué d'un ou de plusieurs couples d'électrodes disposées sur la hauteur de la colonne et reliées à un conductimètre.

Selon une caractéristique supplémentaire, le dispositif comporte un orifice, disposé juste au-dessus du moyen filtrant, destiné à la vidange et au nettoyage de la colonne.

Toujours selon l'invention, le dispositif comporte des moyens informatisés qui assurent le pilotage automatique du système, la visualisation des données brutes et le traitement de ces données pour l'acquisition de paramètres supplémentaires.

Mais l'invention sera encore illustrée, sans être aucunement limitée, par la description suivante d'un mode de réalisation particulier, donné à titre d'exemple et représenté sur les dessins annexés dans lesquels :
- la figure 1 montre de façon schématique l'ensemble du dispositif selon l'invbention ;
- les figures 2 et 3 montrent des courbes de résultats susceptibles d'être obtenues par le dispositif de la figure 1.

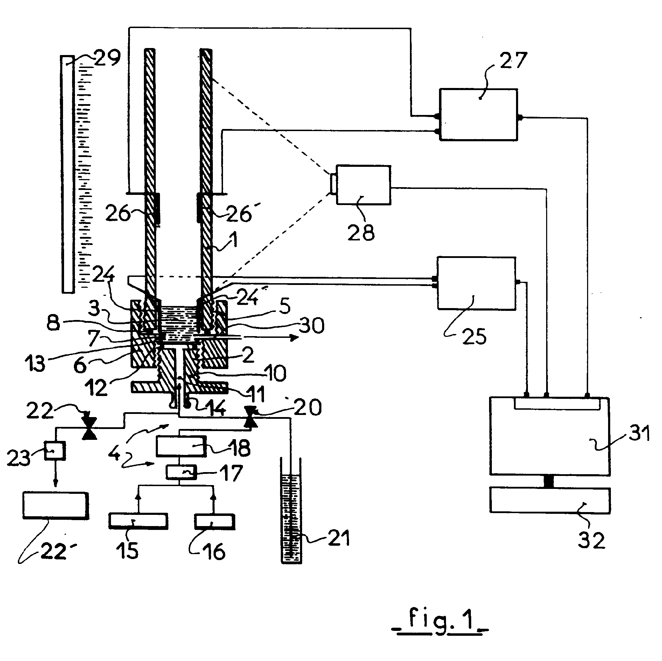

Tel qu'on l'a représenté sur le dessin de la figure 1, le dispositif selon l'invention est constitué d'une colonne d'analyse 1, verticale, munie, à sa base, d'un moyen en forme de filtre 2. Le filtre 2 réalise l'obturation du fond de la colonne 1 pour le cloisonnement du produit 3 à analyser qui se présente sous la forme d'une solution ou d'une suspension. Le filtre 2 permet en outre le passage d'un gaz dont le circuit de distribution 4 est connecté à la partie inférieure de la colonne. Le passage du gaz à travers le filtre 2 est destiné à faire mousser la solution 3 de produit à analyser.

La colonne 1 est associée à un ensemble de capteurs qui permettent la mesure de différents paramètres détaillés plus loin.

La colonne d'analyse 1 est constituée d'un cylindre de verre ou de matériau plastique translucide ; elle est disposée verticalement et comporte, à sa base, un filetage externe 5 qui permet sa solidarisation par vissage sur une embase 6 de réception sensiblement cylindrique. L'embase 6 comporte une collerette interne 7 sur laquelle vient reposer ladite colonne 1 par l'intermédiaire d'un joint d'étanchéité annulaire 8.

La partie inférieure de l'embase 6 reçoit, par vissage, un organe complémentaire 10 percé d'un orifice central 11 destiné à maintenir en place le moyen filtrant 2. Ce dernier peut avantageusement être du type verre fritté, ou autre système à porosité déterminée, choisi en fonction de sa porosité. Le fritté 2 est monté entre l'organe de blocage 10 et la collerette 7 de l'embase 6 par l'intermédiaire de joints annulaires 12 et 13 qui assurent l'étanchéité. La base de l'organe complémentaire 10 comporte un téton circulaire 14 destiné à la connexion du circuit de distribution 4 du flux gazeux.

Le gaz utilisé est fonction du produit que l'on désire analyser ; il peut consister en un flux d'air, d'azote, de gaz carbonique ou de tout autre gaz, obtenu à partir d'un compresseur 15 ou d'une bouteille de stockage 16. Dans les deux cas, le flux gazeux d'origine est contrôlé par un manomètre 17 et passe par un débitmètre 18 qui permet la mesure du volume de gaz délivré. Le fritté 2 qui obture la base de la colonne 1 permet une répartition homogène du flux gazeux qui arrive par l'orifice 11 de façon à faire mousser le produit 3.

Une électrovanne à pincement 20 contrôle la distribution du flux gazeux. En position fermée, elle autorise l'injection de gaz à travers le fritté 2 dans la colonne d'analyse 1 ; en position ouverte, le flux gazeux est stoppé. Lorsque l'électrovanne 20 est dans cette position ouverte, en vue d'arrêter la production de mousse dans la colonne, il est avantageux de prévoir un système de compensation de pression qui permet de garantir l'absence de drainage et de percolation sous ladite colonne 1, à travers le fritté 2. Ce système de contrepression est obtenu en connectant le circuit de distribution 4, par l'intermédiaire de l'électrovanne 20, à une colonne de liquide 21. La hauteur de cette colonne 21 permet de régler la contrepression désirée au niveau du fritté 2.

En outre, sous l'action d'une vanne manuelle 22, le produit 3 peut être aspiré hors de la colonne 1 pour être évacué vers un évier 22′ par exemple. Ce phénomène d'aspiration est avantageusement provoqué par un écoulement d'eau à travers un venturi 23 (trompe à vide) créant ainsi une dépression.

Le circuit de distribution, réalisé par une tuyauterie en plastique, doit être réduit au maximum entre les différents organes pour éviter toute pertubation (perte de charge, étanchéité...).

La colonne d'analyse 1 comporte, à sa base, une paire d'électrodes en platine 24, 24′, reliées à un conductimètre 25. Les électrodes 24, 24′ servent de sonde et sont disposées de telle sorte que le traitement des informations délivrées permette l'obtention du volume de liquide présent dans le fond de la colonne.

Une deuxième paire d'électrodes en platine 26, 26′ est disposée sensiblement au milieu de la colonne 1, au-dessus du niveau de la solution de départ. Cette paire d'électrodes 26, 26′ permet de mesurer la conductivité de la mousse formée ; elle est reliée à un conductimètre 27.

Afin de disposer de différents points de mesure, plusieurs autres couples d'électrodes, identiques à celles 26, 26′ qui viennent d'être décrites, peuvent être disposées sur la hauteur de la colonne.

La hauteur de mousse formée est déterminée par une ou plusieurs cellules photoélectriques, un ou plusieurs capteurs de niveau ou, de préférence, par une caméra linéaire 28.

Cette caméra linéaire 28 est placée face à la colonne 1 afin de permettre la mesure et le suivi automatique de la hauteur de mousse formée. Cette mesure est obtenue par les moyens classiques liés à ce type de matériel ; le contraste pour la lecture est avantageusement amélioré en prévoyant la colonne d'analyse 1 entre la caméra 28 et une source de lumière 29. Connaissant le diamètre de la colonne 1, la mesure de la hauteur de mousse permet d'obtenir, après traitement, la vitesse de formation de la mousse et le volume de mousse formée dans ladite colonne.
A noter que l'embase 6 peut être modifiée pour permettre l'utilisation de colonnes 1 transparentes de diamètre différent ; la caméra 28 peut alors avantageusement être positionnée sur des repères étalonnés qui permettent la détermination de différentes hauteurs de mousse en fonction de la colonne utilisée.

En plus de la mesure de la hauteur de mousse, la caméra 28 peut avantageusement être adaptée et réglée pour permettre la mesure de la densité optique de ladite mousse.

L'embase 6 comporte également un orifice 30 obturable, disposé dans la collerette 7, juste au-dessus du niveau du fritté 2. Cet orifice 30 permet le nettoyage du dispositif, par aspiration de liquide de rinçage au-dessus et à travers le fritté 2.

Les trois types de capteurs : électrodes 24, 24′, électrodes 26, 26′ et caméra 28 permettent l'obtention de mesures brutes absolues qui caractérisent les propriétés moussantes du produit 3 ; ces différentes mesures sont collectées et traitées par des moyens électroniques et informatiques 31, puis affichées sur un écran 32 ; l'ensemble est géré par un logiciel adapté.

L'évolution du volume de liquide suivie par la mesure de la conductivité au niveau des électrodes 24, 24′ permet de mesurer le volume de liquide présent dans la mousse formée.

La hauteur de mousse détectée par la caméra 28 permet l'obtention et le suivi du volume de mousse formée.

L'opacité de la colonne 1, détectée également par la caméra 28 permet en outre de déterminer la "densité optique" (DO) de la mousse.

De plus, et comme on sait déjà le faire, le couple d'électrodes 26, 26′ permet de mesurer et de suivre dans le temps la conductivité de cette mousse.

La mémorisation et le traitement informatique de ces différents paramètres dans le temps, donnent la possibilité de dégager automatiquement tout un ensemble de caractéristiques très précises dont certains exemples vont être définis en liaison avec le processus d'analyse.

La préparation du matériel consiste en une mise sous tension de tous les appareils et en un nettoyage et rinçage de la colonne d'analyse.

Après vérification et réglage du débit de gaz, on verse une quantité déterminée de produit 3 dans la colonne 1. L'électrovanne 20 est ouverte ; la colonne de liquide 21 exerce en permanence une contrepression sur le fritté 2 qui empêche le liquide 3 de descendre dans le conduit 11 de distribution de gaz.

Le système électronique et informatique est réglé en fonction des paramètres variables du dispositif : notamment le diamètre de la colonne 1 ainsi que la détermination du mode d'arrêt d'injection du gaz (en fonction du temps, du volume de mousse obtenue, du volume de liquide utilisé ou du changement de pente d'une des courbes obtenues...).

Lorsque la solution à analyser est introduite dans la colonne, une attente d'environ 30 secondes est nécessaire pour obtenir une stabilisation de la solution. On établit alors une correspondance entre l'affichage informatisé du volume de liquide présent, déterminé par la paire d'électrodes 24, 24′ et la quantité réelle versée par l'utilisateur. Ce réglage est effectué quelle que soit la conductivité du produit à analyser.

L'initialisation du système est réalisée à t = 0.

La phase de prise de mesures commence lors de la fermeture de l'électrovanne 20. Cette fermeture entraîne l'injection du gaz choisi (air, azote, gaz inerte...) dans la colonne 1 et par conséquent, le bullage et la formation de mousse au-dessus du produit 3 en solution ou en suspension.

L'arrêt de l'injection du gaz par l'action de l'électrovanne 20 est fonction du paramètre choisi : temps, volume de liquide, volume de mousse, perte de conductivité de la mousse, volume d'air injecté...

Les prises de mesures par le système informatique et électronique 31, via les conductimètres 25 et 27 et la caméra 28, sont par exemple effectuées toutes les deux secondes à partir de t = 0, pendant 30 à 45 minutes. Ces prises de mesures permettent, dans un premier temps, de déterminer les conditions de formation de la mousse et, ensuite, ses conditions de dégradation. L'arrêt de l'analyse est réalisé soit en fonction du temps passé, du volume de liquide utilisé, du volume de mousse restant, de la conductivité minimum de la mousse ou tout simplement de l'appui sur une touche d'arrêt.

Grâce aux différents capteurs 18, 24-24′, 26-26′ et 28, le dispositif selon l'invention mesure les paramètres suivants : temps, quantité de liquide consommé, conductivité de la mousse, volume de mousse, débit de gaz et "densité optique" de la mousse.

Ces valeurs sont des valeurs absolues directement reliées aux paramètres mesurés ; elles rendent compte en elles-mêmes, en partie, des propriétés de la mousse obtenue et peuvent également être traitées par les moyens informatiques pour caractériser encore plus précisément les propriétés de formation et de dégradation de la mousse, soit ponctuellement, soit dans le temps.

Les moyens informatiques peuvent par exemple permettre la mesure de :
- la densité physique de la mousse, qui correspond au rapport volume de mousse/volume de liquide utilisé ;
- le pouvoir moussant : volume de mousse/volume de gaz ;
- le foisonnement : variation du volume de mousse/variation du volume de gaz ;
- la transparence : fonction du signal caméra ;
- l'homogénéité : fonction de la conductivité de la mousse.

On peut également noter que la mesure de la conductance de la mousse est liée à sa densité ; en effet la conductance est d'autant plus élevée que la mousse est dense : la densité de la mousse étant définie selon la taille des bulles par rapport à la quantité de liquide nécessaire à sa formation.

Le traitement des paramètres mesurés peut également permettre d'obtenir une caractérisation des cinétiques de drainage et de coalescence, qui rendent compte des propriétés de dégradation des mousses.

On peut encore réaliser un suivi dans le temps du volume de la mousse et de ses caractéristiques optiques ou encore faire une analyse des valeurs spécifiques à un produit :
- adsorption maximale de liquide,
- conductivité maximale de la mousse,
- aptitude moussante par mesure de la différence entre le gaz injecté et le volume de mousse.

L'ensemble de ces paramètres (non limitatifs) permet de caractériser la structure et la stabilité de la mousse.

Le traitement informatique permet, à partir des mesures, d'obtenir, pendant le temps de l'expérience, différents types de courbes dont : la quantité de liquide dans la mousse, le niveau de liquide, le volume de mousse, la conductivité, leurs transformés, logarithme ou exponentielle, les variations de leur pente, le lissage des courbes expérimentales, les. régressions qui peuvent en être tirées, etc...

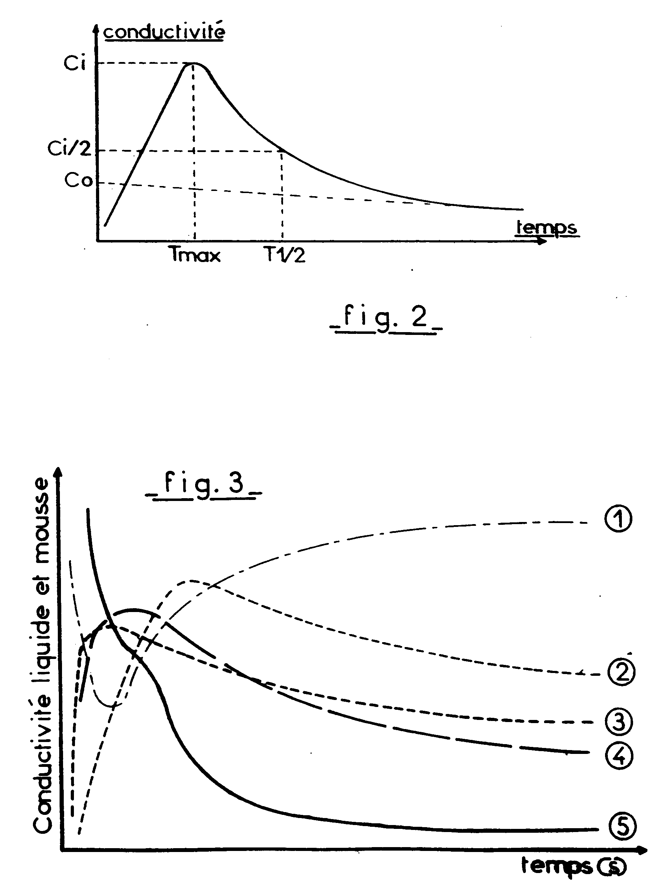

On a représenté sur la figure 2, un exemple possible de courbe montrant la conductivité d'une mousse dans le temps.

Cette courbe permet de déterminer :
- la capacité moussante : Ci
- la stabilité de la mousse : t½ correspondant à Ci/2
- l'indice de stabilité : Co x (Δ T/ Δ C)

On a représenté sur la figure 3, différents exemples de courbes que l'on peut obtenir avec le dispositif selon l'invention :
Courbe 1 : cinétique du liquide dans la colonne,
Courbe 2 : évolution du volume de mousse,
Courbe 3 : Log du liquide dans la mousse,
Courbe 4 : Log conductivité de la mousse,
Courbe 5 : densité de la mousse.

Le dispositif selon l'invention permet de mesurer toute une gamme de valeurs qui rendent compte de façon complète des propriétés moussantes de produits en solution ou en suspension.

Le pilotage informatique permet de choisir avec une grande précision les systèmes qui commandent la formation de la mousse : le temps, le volume, le liquide, le changement de pente...

Ce système est très polyvalent quant au volume de liquide utilisé (dépend de la géométrie des cellules de mousse) ; il permet une utilisation de volumes de solution compris entre 5 et 25 ml.

Ce type de dispositif trouve une application particulièrement intéressante dans le domaine de la cosmétologie ou en agroalimentaire, par exemple pour déterminer les caractéristiques moussantes de protéines. Tout laboratoire de recherche et de développement peut l'utiliser pour caractériser des matières premières et des produits finis.

Les signes de référence insérés après les caractéristiques techniques mentionnées dans les revendications ont pour seul but de faciliter la compréhension de ces dernières et n'en limitent aucunement la portée.

## Revendications

1. Dispositif de caractérisation des propriétés moussantes d'un produit au moins partiellement soluble, lequel dispositif comporte une colonne d'analyse (1), transparente, munie, à sa base, d'un moyen permettant l'incorporation d'un flux de gaz et sa répartition de façon homogène pour faire mousser le produit (3) placé dans le bas de ladite colonne d'analyse (1), caractérisé en ce qu'il comporte :
- un système (28) de mesure automatique de la hauteur de mousse formée dans la colonne (1),
- un système (24, 24′, 25) de mesure automatique du volume de produit (3) utilisé pour former la mousse,
- des moyens qui permettent de visualiser et/ou de traiter les données brutes recueillies.

2. Dispositif selon la revendication 1, caractérisé en ce que le moyen qui permet l'incorporation d'un flux de gaz et sa répartition de façon homogène consiste en un filtre poreux (2).

3. Dispositif selon l'une quelconque des revendications 1 ou 2, caractérisé en ce que le système de mesure de la hauteur de mousse consiste en une caméra linéaire (28).

4. Dispositif selon l'une quelconque des revendications 1 à 3, caractérisé en ce que le système de mesure du volume de produit (3) utilisé consiste en une paire d'électrodes (24, 24′) disposées dans le fond de la colonne (1) et reliées à un conductimètre (25).

5. Dispositif selon l'une quelconque des revendications 2 à 4, caractérisé en ce qu'il comporte un moyen de distribution du flux gazeux en forme de vanne (20) dont la fermeture permet l'injection de gaz à travers le filtre (2) et dont l'ouverture réalise la connexion avec un dispositif en forme de colonne de liquide (21) permettant d'assurer une contrepression à travers le filtre (2) afin d'éviter la percolation et le drainage du produit (3) au travers dudit filtre (2).

6. Dispositif selon l'une quelconque des revendications 1 à 5, caractérisé en ce qu'il comporte un système de mesure (18) de la quantité de gaz insufflé.

7. Dispositif selon l'une quelconque des revendications 1 à 6, caractérisé en ce qu'il comporte un système (28) de mesure de l'opacité de la mousse formée.

8. Dispositif selon l'une quelconque des revendications 1 à 7, caractérisé en ce qu'il comporte un système de mesure de la conductivité de la mousse, constitué d'au moins un couple d'électrodes (26, 26′) disposées sur la hauteur de la colonne (1) et reliées à un conductimètre (27).

9. Dispositif selon l'une quelconque des revendications 2 à 8, caractérisé en ce qu'il comporte un orifice (30) disposé juste au-dessus du filtre (2), destiné à la vidange et au nettoyage du dispositif.

10. Dispositif selon l'une quelconque des revedications 1 à 9, caractérisé en ce qu'il comporte des moyens informatisés qui permettent le pilotage automatique du système, la visualisation des données brutes et le traitement de ces données pour l'acquisition de paramètres supplémentaires.

## Patentansprüche

1. Vorrichtung zur Charakterisierung der Schäumungseigenschaften eines wenigstens teilweise löslichen Produkts, wobei die Vorrichtung eine transparente Analysesäule (1) umfaßt, die auf ihrem Boden mit einer Einrichtung versehen ist, die das Einleiten eines Gasstroms und dessen homogene Verteilung erlaubt, um das in den unteren Teil der Analysesäule (1) gebrachte Produkt (3) schäumen zu lassen, dadurch gekennzeichnet, daß sie umfaßt:
- ein System (28) zur automatischen Messung der Höhe des in der Säule (1) gebildeten Schaumes,
- ein System (24, 24′, 25) zur automatischen Messung des Volumens des zur Bildung des Schaumes verwendeten Produkts (3),
- Vorrichtungen, die es erlauben, die gewonnenen Rohdaten sichtbar zu machen und/oder zu verarbeiten.

2. Vorrichtung gemäß Anspruch 1, dadurch gekennzeichnet, daß die Einrichtung, die das Einleiten eines Gasstroms und dessen homogene Verteilung erlaubt, aus einem porösen Filter (2) besteht.

3. Vorrichtung gemäß einem der Ansprüche 1 oder 2, dadurch gekennzeichnet, daß das System zur Messung der Höhe des Schaumes aus einer linearen Kamera (28) besteht.

4. Vorrichtung gemäß einem der Ansprüche 1 bis 3, dadurch gekennzeichnet, daß das verwendete System zur Messung des Volumens des Produkts (3) aus einem Paar von Elektroden (24, 24′) besteht, die auf dem Boden der Säule (1) angebracht und mit einem Konduktometer (25) verbunden sind.

5. Vorrichtung gemäß einem der Ansprüche 2 bis 4, dadurch gekennzeichnet, daß sie eine Einrichtung zur Verteilung des Gasstroms in Form eines Schiebers (20) umfaßt, der, wenn er geschlossen ist, das Einleiten von Gas durch das Filter (2) erlaubt, und der beim Öffnen eine Verbindung mit einer Vorrichtung in Form einer Flüssigkeitssäule (21) schafft, die es erlaubt, einen Gegendruck durch das Filter (2) zu gewährleisten, um das Durchsickern und Auslaufen des Produkts (3) durch das Filter (2) zu vermeiden.

6. Vorrichtung gemäß einem der Ansprüche 1 bis 5, dadurch gekennzeichnet, daß sie ein System (18) zur Messung der Menge des eingeblasenen Gases umfaßt.

7. Vorrichtung gemäß einem der Ansprüche 1 bis 6, dadurch gekennzeichnet, daß sie ein System (28) zur Messung der Lichtundurchlässigkeit des gebildeten Schaums umfaßt.

8. Vorrichtung gemäß einem der Ansprüche 1 bis 7, dadurch gekennzeichnet, daß sie ein System zur Messung der Leitfähigkeit des Schaums umfaßt, das aus wenigstens einem Paar von Elektroden (26, 26′) besteht, die auf der Höhe der Säule (1) angebracht und mit einem Konduktometer (27) verbunden sind.

9. Vorrichtung gemäß einem der Ansprüche 2 bis 8, dadurch gekennzeichnet, daß sie eine Öffnung (30) umfaßt, die gerade oberhalb des Filters (2) angebracht ist und für die Entleerung und Reinigung der Vorrichtung bestimmt ist.

10. Vorrichtung gemäß einem der Ansprüche 1 bis 9, dadurch gekennzeichnet, daß sie Vorrichtungen zur Datenverarbeitung umfaßt, die eine automatische Steuerung des Systems, die Sichtbarmachung der Rohdaten und die Verarbeitung dieser Daten zur Erlangung zusätzlicher Parameter erlauben.

## Claims

1. Characterization device of the foaming properties of a product which is at least partially soluble, said device comprising an analysis column (1), transparent, fitted at its base with a means allowing to introduce a gas flow and to distribute it homogeneously to cause the foaming of the product (3), located at the bottom of said analysis column (1), characterized in that it comprises:
- an automatic measuring system (28) of the height of foam formed in the column (1),
- an automatic measuring system (24, 24′, 25) of the volume of product (3) used to form the foam,
- means which enable to visualize and/or to process the obtained raw data.

2. Device according to claim 1, characterized in that the means which enables to introduce a gas flow and its homogeneous distribution comprises a porous filter (2).

3. Device according to any one of claims 1 or 2, characterized in that the measuring system of the foam height comprises a linear camera (28).

4. Device according to any one of claims 1 to 3, characterized in that the measuring system of the volume of product (3) used comprises a pair of electrodes (24, 24′) arranged at the bottom of the column (1) and connected to a conductimeter (25).

5. Device according to any one of claims 2 to 4, characterized in that it comprises a means of distribution of the gas flow in the form of a valve (20) whose closing enables injection of gas through the filter (2) and whose opening ensures connection with a device in the form of a column of liquid (21) enabling to provide back pressure through the filter (2) in order to avoid percolation and drainage of the product (3) through said filter (2).

6. Device according to any one of claims 1 to 5, characterized in that it comprises a measuring system (18) of the quantity of gas injected.

7. Device according to any one of claims 1 to 6, characterized in that it comprises an opacity measuring system (28) of the foam formed.

8. Device according to any one of claims 1 to 7, characterized in that it comprises a conductivity measuring system of the foam, comprising at least a couple of electrodes (26, 26′) arranged along the height of the column (1) and connected to a conductimeter (27).

9. Device according to one of claims 2 to 8, characterized in that it comprises a hole (30) arranged immediately above the filter (2), designed for draining and cleaning the device.

10. Device according to any one of claims 1 to 9, characterized in that it comprises computerized means enabling automatic monitoring of the system, viewing raw data and processing this data in order to obtain additional parameters.
